# EUROPEAN PATENT SPECIFICATION

(11) **EP 1 752 104 B1**
(45) Date of publication and mention of the grant of the patent: **25.03.2009**
(21) Application number: 06253940.8
(22) Date of filing: 27.07.2006
(51) Int. Cl.: A61B 17/11

(54) **Electroactive polymer-based tissue apposition device and method of use**
Chirurgische Appositionsvorrichtung mit elektroaktivem Polymer
Dispositif d'apposition chirurgale à base d'un polymèr électroactif

(30) Priority: 28.07.2005 US 161264
(43) Date of publication of application: 14.02.2007
(73) Proprietor: ETHICON ENDO-SURGERY, INC., Cincinnati, Ohio 45242 (US)
(72) Inventor: Ortiz, Mark S., Milford, OH 45150 (US); Freeman, Lynetta, West Chester OH 45069 (US)
(74) Representative: Tunstall, Christopher Stephen

(56) References cited:
- US-A- 2003 171 775
- US-A1- 2003 229 332
- US-A1- 2003 236 531
- US-A1- 2004 167 546
- US-A1- 2005 113 892
- US-B1- 6 249 076

## Description

### FIELD OF THE INVENTION

The present invention relates broadly to surgical devices, and in particular to methods and devices for positioning tissues in apposition to each other.

### BACKGROUND OF THE INVENTION

In cases of severe obesity, patients can undergo various types of surgical procedures to tie off, staple, or bypass portions of the stomach and gastrointestinal tract (e.g., large intestine or small intestine). These procedures can reduce the amount of food desired and ingested by the patient, thereby causing the patient to lose weight.

One surgical procedure, known as a Roux-En-Y gastric bypass, creates a permanent surgical reduction of a patient's stomach volume and a bypass of the patient's intestine. In the procedure, the stomach is separated into a smaller, upper stomach pouch and a larger, lower stomach pouch, such as by using a stapling device. A segment of the patient's small intestine (e.g., a segment distal of the duodenum or proximal of the jejunum) is then brought from the lower abdomen and joined with the upper stomach pouch created through a half-inch opening, or stoma, in the stomach pouch and small intestine. This segment of the small intestine, known as the "Roux loop,''' carries food from the upper stomach pouch to the remainder of the intestines, where the food is digested. The remaining lower stomach pouch and the attached segment of duodenum are then reconnected to form another anastomotic connection to the Roux loop at a location approximately 50-150 cm (1.6-4.9 ft) from the stoma, typically using a stapling instrument. From this connection, digestive juices from the bypassed stomach (e.g., the lower stomach pouch), pancreas, and liver enter the jejunum or ileum to aid in digestion. The relatively small size of the upper stomach pouch therefore reduces the amount of food that the patient can eat at one time, thereby leading to weight loss in the patient.

In the Roux-En-Y gastric bypass, many techniques can be used to orient the small intestine relative to the upper stomach pouch. Certain conventional instruments include a flexible shaft having proximal and distal balloons used to engage the walls of the upper stomach pouch and small intestine. In particular, the distal balloon can be inserted within an opening of the small intestine and expanded to contact the small intestine wall and the proximal balloon can be inserted within the upper stomach pouch and inflated to contact the upper stomach pouch wall. As the flexible shaft is manipulated within the patient (e.g., is pushed distally or pulled proximally within the patient), the balloons position the upper stomach pouch and the small intestine in proximity to each other. A tissue coupling device can be oriented at the juncture of the upper stomach pouch and small intestine to apply staples or sutures to couple the pouch to the small intestine.

US-A1-2003/71775 discloses a tissue apposition device upon which the two part form of appended claim 1 is based. It may use balloons or mechanical expansion means.

While the use of an instrument having a flexible shaft and balloons can be an effective mechanism to position the upper stomach pouch and the small intestine or duodenum in relation to one another, difficulty can be encountered by use of such an instrument. For example, during operation of the instrument the balloons can potentially leak and become deflated, such as caused by puncturing or over inflation of the balloons, thereby limiting the ability for the instrument to control the relative positioning of the tissues. Additionally, the flexibility of the shaft can interfere with positioning of the balloons.

Accordingly, there is a need for improved methods and devices for positioning tissues in apposition to each other.

### SUMMARY OF THE INVENTION

The present invention generally provides devices for positioning tissues in apposition to each other, as in appended claim 1.

The elongate member can have a variety of configurations, but in one embodiment the elongate member can include a fixed portion and a moveable portion moveably coupled to the fixed portion and having an expandable element coupled thereto. While various techniques can be used to moveably couple the moveable portion and the fixed portion, in one embodiment the moveable portion can be slidably disposed within an inner lumen formed in the fixed portion. The positioning element can be coupled to and can extend between the moveable portion and the fixed portion such that activation of the positioning element slides the moveable portion relative to the fixed portion, thereby moving the expandable element axially. In an exemplary embodiment, the positioning element can be an electroactive polymer cord that axially contracts when energy is delivered thereto to move the expandable element. In another embodiment, at least a portion of the elongate member can be flexible to allow movement between the fixed portion and the moveable portion. The positioning element can be coupled to the flexible portion and it can be adapted to axially contract the flexible portion upon energy delivery thereto to axially move the at least one expandable element relative to the elongate member. In an exemplary embodiment, the positioning element can be at least one electroactive polymer composite.

In another embodiment, a tissue apposition device is provided having an elongate member, at least one electrically actuatable expandable element coupled to the elongate member and adapted to engage tissue, and an electrically actuatable positioning element coupled to the elongate member and adapted to axially move the at least one electrically expandable element relative to the elongate member. In an exemplary embodiment, the device includes first and second electrically actuatable expandable elements coupled to the elongate shaft and spaced a distance apart from one another, the electrically actuatable positioning element is adapted to move the first and second electrically actuatable expandable elements toward one another.

Methods for positioning tissues in apposition to one another are also disclosed. The method can include inserting an elongate shaft through a first tissue and a second tissue, and electrically actuating at least one electroactive polymer actuator coupled to the elongate shaft to engage and move the second tissue toward the first tissue. Electrically actuating at least one electroactive polymer actuator can include electrically expanding a first electroactive polymer coupled to the elongate member and adapted to engage the second tissue, and electrically actuating a second electroactive polymer coupled to the elongate member to axially move the first electroactive polymer relative to the elongate shaft. The second electroactive polymer can be coupled to a moveable portion of the elongate shaft such that electrically actuating the second electroactive polymer slides the moveable portion relative to a fixed portion of the elongate shaft. The method can also include electrically actuating a second electroactive polymer coupled to the elongate shaft to engage the first tissue.

### BRIEF DESCRIPTION OF THE DRAWINGS

The invention will be more fully understood from the following detailed description taken in conjunction with the accompanying drawings, in which:
FIG. 1A is a perspective view of one exemplary embodiment of a tissue apposition device;
FIG. 1B is a cross-sectional view of the tissue apposition device of FIG. 1A showing a positioning element coupled to a fixed portion and a moveable portion of an elongate shaft;
FIG. 1C is a an enlarged cross-sectional view of a distal portion of the tissue apposition device of FIG. 1B;
FIG. 2A is a cross-sectional view of a prior art fiber bundle type electroactive polymer (EAP) actuator;
FIG. 2B is a radial cross-sectional view of the prior art actuator shown in FIG. 2A;
FIG. 3A is a cross-sectional view of a prior art laminate type EAP actuator having multiple EAP composite layers;
FIG. 3B is a perspective view of one of the composite layers of the prior art actuator shown in FIG. 3A;
FIG. 4 is a perspective view of another embodiment of a tissue apposition device having positioning actuators disposed on opposed sides of a flexible portion of an elongate shaft that is configured to move first and second expandable elements relative to one another;
FIG. 5A is an illustration showing the tissue apposition device of FIGS. 1A-1C inserted through proximal and distal tissues;
FIG. 5B is an illustration showing a distal expandable element on the tissue apposition device of FIG. 5A expanded to engage the distal tissue;
FIG. 5C is an illustration showing a positioning actuator of the tissue apposition device of FIG. 5B axially contracted to axially move the second expandable element and distal tissue toward the proximal tissue;
FIG. 5D is an illustration showing a first expandable element of the tissue apposition device of FIG. 5C expanded to engage the proximal tissue;
FIG. 5E is an illustration showing tissue fasteners implanted in the proximal and distal tissues around the tissue apposition device of FIG. 5D; and
FIG. 5F is a perspective view of the tissue apposition device of FIG. 5E showing the distal element and the proximal element contracted to remove the tissue apposition device from the joined tissues.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are nonlimiting exemplary embodiments and that the scope of the present invention is defined solely by the claims. The features illustrated or described in connection with one exemplary embodiment may be combined with features of other embodiments. Such modifications and variations are intended to be included within the scope of the present invention.

The present invention generally provides devices for positioning tissues in apposition to each other, such as during a gastric bypass procedure. In one exemplary embodiment, a tissue apposition device is provided having a flexible shaft with at least one expandable element coupled thereto, and a positioning element coupled to the elongate shaft and adapted to move the expandable element(s) axially to move tissue engaged by the expandable element(s). In an exemplary embodiment, the positioning element and/or the expandable element(s) can be electrically actuatable such that they are adapted to change dimensionally, e.g., expand in one direction and contract in an opposite direction, to engage and move tissues. Thus, for example, the expandable element(s) can be adapted to radially expand to engage tissue, and/or the positioning element can be adapted to axially contract to move the expandable element(s) axially, thereby moving the tissue axially. The use of electrically actuatable positioning elements can eliminate the need to deliver a force through the length of the flexible shaft, and the use of electrically actuatable expandable elements can allow for an easier, more controlled technique for engage tissue. In certain exemplary embodiments, two expandable elements can be used to engage first and second tissues to move the tissues into apposition to one another. A person skilled in the art will appreciate that the device can include any combination of electrically actuatable members and non-electrically actuatable members, and that the device can be used for a variety of purposes other than to move tissues into apposition with one another.

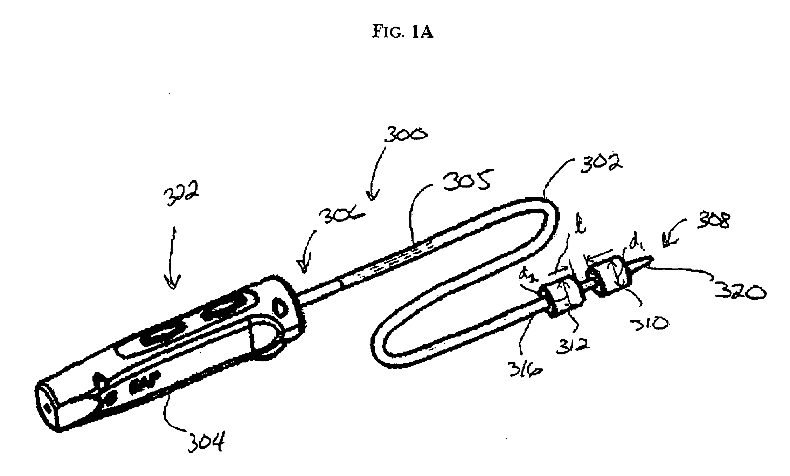

FIGS. 1A-1C illustrate one exemplary embodiment of a tissue apposition device 300. As shown, the device 300 generally includes an elongate member or shaft 302, first and second expandable elements 310, 312 coupled to the elongate shaft 302, and a positioning element 314 coupled to the elongate shaft 302 and adapted to move the first and/or second expandable elements 310, 312 relative to one another. In use, the expandable elements 310, 312 can be positioned on opposed sides of first and second tissue surfaces, and one or both of the expandable elements 310, 312 can be expanded to engage the tissue. The positioning element 314 can then be activated to move one or both of the expandable elements 310, 312 toward one another, thereby positioning the tissues in apposition to one another.

The elongate shaft 302 can have a variety of configurations, but in the illustrated embodiment it has a generally elongate shape with proximal and distal ends 306, 308. The proximal end 306 can be coupled to a handle 304, as shown in FIG. 1A, to facilitate grasping and manipulation of the device 300. The handle 304 can also include other components to facilitate operation of the device, as will be discussed in more detail below. The distal end 308 of the elongate shaft 302 can be adapted to insert into or through tissue, and it can include features, such as a tapered tip 320, to facilitate insertion. The elongate shaft 302 can also be formed from a variety of materials but in one exemplary embodiment it can be formed from a substantially flexible material that allows the elongate member 302 to bend or follow a general curvature of a lumen in a patient's body. In an exemplary embodiment, as shown in FIG. 1A, the elongate shaft 302 defines an inner lumen 305 extending through at least a portion of the elongate shaft 302, and optionally through the entire length of the elongate shaft 302 and the handle 304 of the device 300, depending on the intended use of the lumen 305. The lumen 305 can be configured to receive or contain various components, such as an imaging device, e.g., a camera, or a variety of other medical devices, such as flexible graspers and other surgical tools. The lumen 305 can also be configured to deliver or remove fluids or other materials to a surgical site.

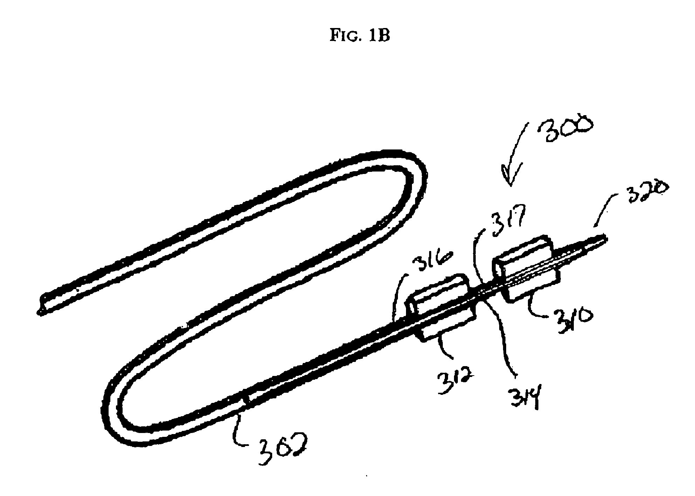

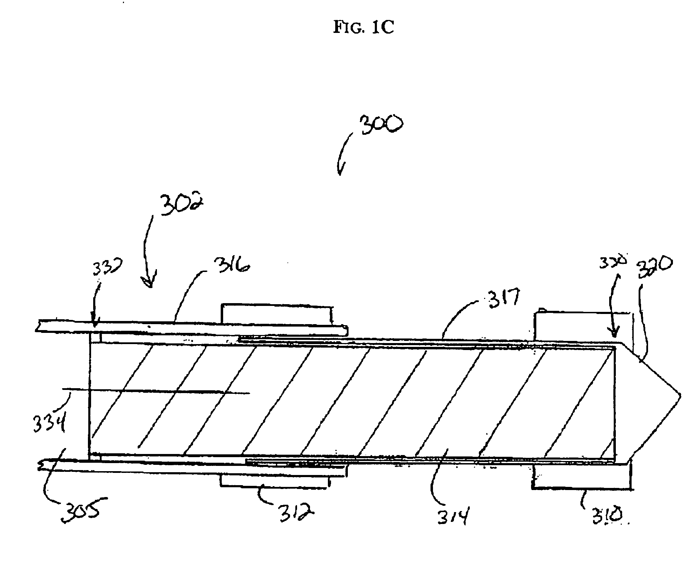

As shown in more detail in FIGS. 1B and 1C, the elongate shaft 302 can also include a fixed portion 316 having the second expandable element 312 coupled thereto, and a moveable portion 317 having the first expandable element 310 coupled thereto to allow relative movement of the first and second expandable elements 310, 312. While various techniques can be used to provide fixed and movable portions 316, 317, in the illustrated embodiment the fixed portion 316 is attached to and extends from the handle 304, and the moveable portion 317 is in the form of a generally tubular structure that is slidably disposed within and extends distally from the lumen 305 defined by the fixed portion 316. As a result, the moveable portion 317 can slide along an axis 334 of the elongate shaft 302 relative to the fixed portion 316 between a retracted, proximal-most position in which the first and second expandable elements 310, 312 are positioned substantially adjacent to one another, and an extended, distal-most position in which the first and second expandable elements 310, 312 are spaced a distance apart from one another. A person skilled in the art will appreciate that a variety of other techniques can be used to allow relative movement between the first and second expandable elements 310, 312. As will be explained in more detail below, in another embodiment the moveable portion 316 and the fixed portion 317 can be moveably coupled to each other by a flexible portion formed therebetween along a portion of the elongate member 302.

As indicated above, the illustrated device 300 also includes first and second expandable elements 310, 312 and a positioning element 314 for moving the first and/or second expandable elements 310, 312 relative to each other. In one exemplary embodiment, at least one of these elements 310, 312, 314 can be formed from an electrically actuatable material that can change dimensions in response to application of electrical energy to the material. The electrically actuatable material forming any one or more of the elements 310, 312, 314 can couple to a variety of electrical sources to facilitate such dimensional change. For example, in one embodiment, an energy source, such as a battery, can be disposed within the handle 304 for delivering energy to the electrically actuatable material. Alternatively, the handle 304 can be adapted to couple to an energy source, such as an electrical outlet. The handle 304 can also include one or more controllers 322, as shown in FIG. 1A, configured to regulate an amount of electrical energy provided by an energy source to the electrically actuatable material to control a corresponding degree of geometric or dimensional change of the electrically actuatable material. For example, by allowing a relatively small amount of voltage to pass from the power source to the electrically actuatable material, the controller 322 can cause the electrically actuatable material to change dimensions by a relatively small degree. Alternatively, by allowing a relatively large amount of voltage to pass from the power source to the electrically actuatable material, the controller 322 can cause the electrically actuatable material to change dimensions by a relatively large degree, as will be discussed in more detail below.

According to the claimed invention, each of the first and second expandable elements 310, 312 and the positioning element 314 can be formed from an electrically actuatable material, in one exemplary embodiment, at least one of the elements 310, 312, 314 can be formed from an electroactive polymer material. Electroactive polymers (EAPs), also referred to as artificial muscles, are materials that exhibit piezoelectric, pyroelectric, or electrostrictive properties in response to electrical or mechanical fields. In particular, EAPs are a set of conductive doped polymers that change shape when an electrical voltage is applied. The conductive polymer can be paired with some form of ionic fluid or gel using electrodes. Upon application of a voltage potential to the electrodes, a flow of ions from the fluid/gel into or out of the conductive polymer can induce a shape change of the polymer. Typically, a voltage potential in the range of about 1V to 4kV can be applied depending on the particular polymer and ionic fluid or gel used. It is important to note that EAPs do not change volume when energized, rather they merely expand in one direction and contract in a transverse direction.

One of the main advantages of EAPs is the possibility to electrically control and fine-tune their behavior and properties. EAPs can be deformed repetitively by applying external voltage across the EAP, and they can quickly recover their original configuration upon reversing the polarity of the applied voltage. Specific polymers can be selected to create different kinds of moving structures, including expanding, linear moving, and bending structures. The EAPs can also be paired to mechanical mechanisms, such as springs or flexible plates, to change the effect of the EAP on the mechanical mechanism when voltage is applied to the EAP. The amount of voltage delivered to the EAP can also correspond to the amount of movement or change in dimension that occurs, and thus energy delivery can be controlled to effect a desired amount of change.

There are two basic types of EAPs and multiple configurations for each type. The first type is a fiber bundle that can consist of numerous fibers bundled together to work in cooperation. The fibers typically have a size of about 30-50 microns. These fibers may be woven into the bundle much like textiles and they are often referred to as EAP yarn. In use, the mechanical configuration of the EAP determines the EAP actuator and its capabilities for motion. For example, the EAP may be formed into long strands and wrapped around a single central electrode. A flexible exterior outer sheath will form the other electrode for the actuator as well as contain the ionic fluid necessary for the function of the device. When voltage is applied thereto, the EAP will swell causing the strands to contract or shorten. An example of a commercially available fiber EAP material is manufactured by Santa Fe Science and Technology and sold as PANION™ fiber and described in U.S. Pat. No. 6,667,825, which is hereby incorporated by reference in its entirety.

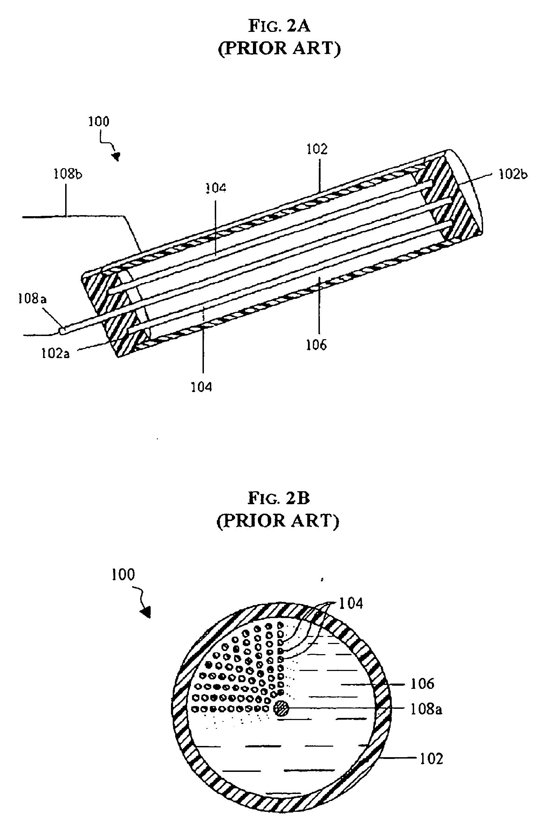

FIGS. 2A and 2B illustrate one exemplary embodiment of an EAP actuator 100 formed from a fiber bundle. As shown, the actuator 100 generally includes a flexible conductive outer sheath 102 that is in the form of an elongate cylindrical member having opposed insulative end caps 102a, 102b formed thereon. The conductive outer sheath 102 can, however, have a variety of other shapes and sizes depending on the intended use. As is further shown, the conductive outer sheath 102 is coupled to a return electrode 108a, and an energy delivering electrode 108b extends through one of the insulative end caps, e.g., end cap 102a, through the inner lumen of the conductive outer sheath 102, and into the opposed insulative end cap, e.g., end cap 102b. The energy delivering electrode 108b can be, for example, a platinum cathode wire. The conductive outer sheath 102 can also include an ionic fluid or gel 106 disposed therein for transferring energy from the energy delivering electrode 108b to the EAP fibers 104, which are disposed within the outer sheath 102. In particular, several EAP fibers 104 are arranged in parallel and extend between and into each end cap 102a, 120b. As noted above, the fibers 104 can be arranged in various orientations to provide a desired outcome, e.g., radial expansion or contraction, or bending movement. In use, energy can be delivered to the actuator 100 through the active energy delivery electrode 108b and the conductive outer sheath 102 (anode). The energy will cause the ions in the ionic fluid to enter into the EAP fibers 104, thereby causing the fibers 104 to expand in one direction, e.g., radially such that an outer diameter of each fiber 104 increases, and to contract in a transverse direction, e.g., axially such that a length of the fibers decreases. As a result, the end caps 102a, 120b will be pulled toward one another, thereby contracting and decreasing the length of the flexible outer sheath 102.

Another type of EAP is a laminate structure, which consists of one or more layers of an EAP, a layer of ionic gel or fluid disposed between each layer of EAP, and one or more flexible conductive plates attached to the structure, such as a positive plate electrode and a negative plate electrode. When a voltage is applied, the laminate structure expands in one direction and contracts in a transverse or perpendicular direction, thereby causing the flexible plate(s) coupled thereto to shorten or lengthen, or to bend or flex, depending on the configuration of the EAP relative to the flexible plate(s). An example of a commercially available laminate EAP material is manufactured by Artificial Muscle Inc, a division of SRI Laboratories. Plate EAP material, referred to as thin film EAP, is also available from EAMEX of Japan.

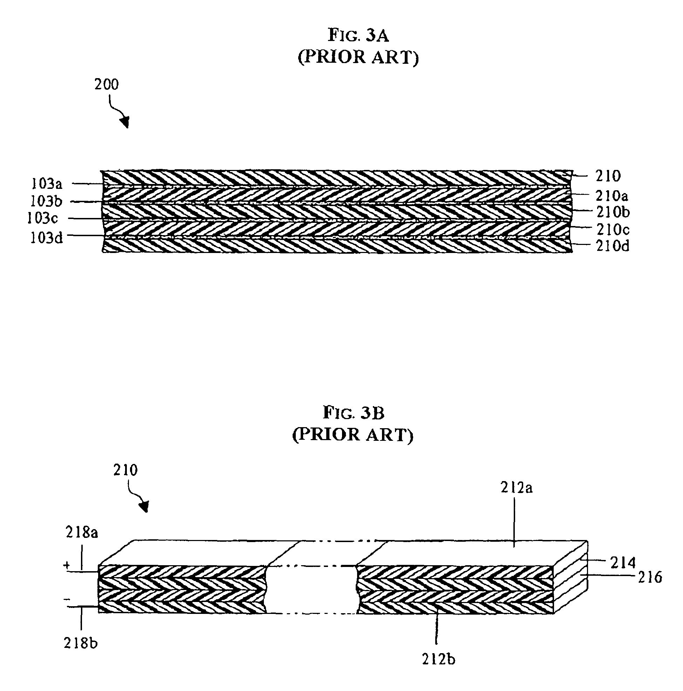

FIGS. 3A and 3B illustrate an exemplary configuration of an EAP actuator 200 formed from a laminate. Referring first to FIG. 3A, the actuator 200 can include multiple layers, e.g., five layers 210, 210a, 210b, 210c, 210d are shown, of a laminate EAP composite that are affixed to one another by adhesive layers 103a, 103b, 103c, 103d disposed therebetween. One of the layers, i.e., layer 210, is shown in more detail in FIG. 3B, and as shown the layer 210 includes a first flexible conductive plate 212a, an EAP layer 214, an ionic gel layer 216, and a second flexible conductive plate 212b, all of which are attached to one another to form a laminate composite. The composite can also include an energy delivering electrode 218a and a return electrode 218b coupled to the flexible conductive plates 212a, 212b, as further shown in FIG. 3B. In use, energy can be delivered to the actuator 200 through the active energy delivering electrode 218a. The energy will cause the ions in the ionic gel layer 216 to enter into the EAP layer 214, thereby causing the layer 214 to expand in one direction and to contract in a transverse direction. As a result, the flexible plates 212a, 212b will be forced to flex or bend, or to otherwise change shape with the EAP layer 214.

Returning to FIGS. 1A-1C, either type of actuator can be used to form one or both of the expandable elements 310, 312, however in an exemplary embodiment the expandable elements 310, 312 are formed using an EAP laminate, or a composite EAP formed from multiple laminate layers. The first and second expandable elements 310, 312 can be formed by rolling the EAP actuator around the elongate shaft 302 of the device 300. The actuator can be mated to the shaft using an adhesive or other mating technique. While not shown, the expandable elements 310, 312 can optionally be disposed within the inner lumen 305 of the elongate shaft 302 and/or embedded within the walls of the elongate shaft 302, or alternatively the expandable elements 310, 312 can be formed integrally with the elongate shaft 302. Moreover, while the device 300 is shown having two expandable elements 310, 312, the device 300 can include any number of expandable elements located in any position relative to the longitudinal axis 334 of the elongate shaft 302.

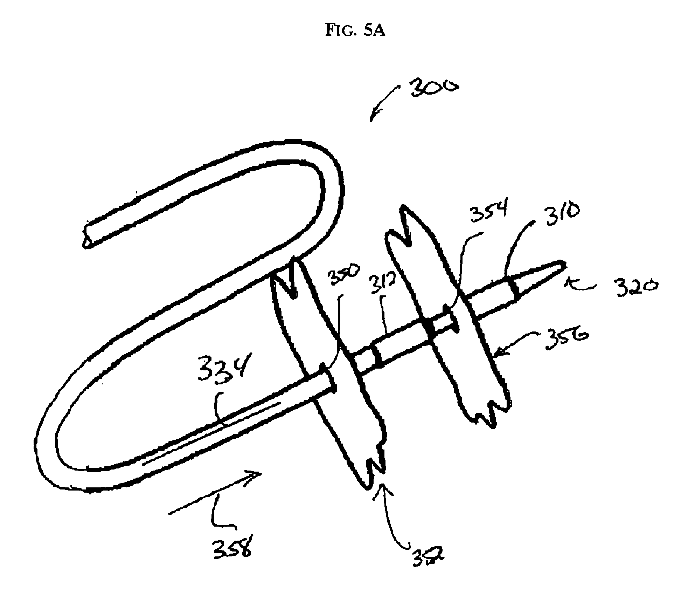

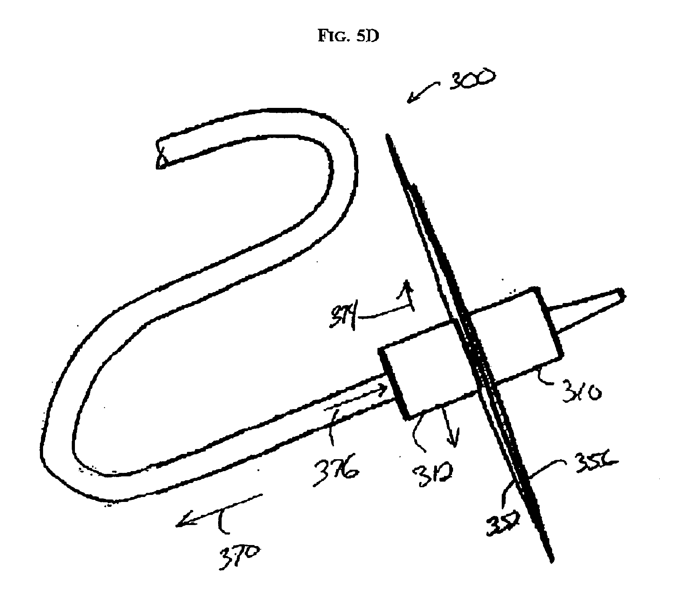

In use, the orientation of the EAP actuators can be configured to allow the first and second expandable elements 310, 312 to radially expand and axially contract upon application of electrical energy to the expandable elements 310, 312. In particular, when energy is delivered to the first and second expandable elements 310, 312, the diameter d₁, d₂ of each expandable element 310, 312 can increase from an unexpanded position (FIG. 5A) to an expanded position, as shown in FIG. 1A. Based upon such a change in geometry, the expandable elements 310, 312 can be configured to engage body tissues or organs oriented in proximity to the elements 310, 312. A person skilled in the art will appreciate that various techniques can be used to deliver energy to the expandable elements 310, 312. For example, the expandable elements 310, 312 can be coupled to a return electrode and a delivery electrode that is adapted to communicate energy from a power source to the actuator. The electrodes can extend through the inner lumen in the elongate shaft 302, be embedded in the sidewalls of the elongate shaft 302, or they can extend along an external surface of the elongate shaft 302.

The positioning element 314 can also be formed using either type of EAP, however in one exemplary embodiment the positioning element 314 is formed using the fiber bundle type EAP, which is also referred to herein as a cord EAP. In the embodiment shown in FIGS. 1B and 1C, the cord type EAP positioning element 314 can couple to the elongate shaft 302 between the moveable portion 317 and the fixed portion 316 of the shaft 302. In particular, while the positioning element 314 can couple to an external surface of the elongate shaft 302 or it can be embedded within a portion of the elongate shaft 302, in an exemplary embodiment the cord type EAP positioning element 314 is disposed within the lumen 305 of the shaft 302 and it is coupled to and extends between a distal end 330 of the moveable portion 317 and a sidewall 332 of the inner lumen 305 of the fixed portion 316. The location of the distal end 330 of the positioning element 314 within the lumen 305 can vary based upon a desired amount of movement of the moveable portion 317 relative to the fixed portion 316 of the elongate shaft 302. For example, wherein the first expandable element 310 is positioned a predetermined distance apart from the second expandable element 312 when the moveable portion 317 is in an extended, distal-most position, the positioning element 314 can couple to the sidewall 332 of the inner lumen 305 of the fixed portion 316 at a location that is the predetermined distance away from the distal-most end of the fixed portion 316. The positioning element 314 can, however, extend through a greater portion of the elongate shaft 302, or it can extend through the entire length of the elongate shaft 302. A person skilled in the art will appreciate that the positioning element 314 can be formed from non-electrically actuatable materials. For example, the positioning element can be in the form of an elongate cord or shaft extending through the device and adapted to be pulled in a proximal direction to move the expandable elements 310, 312 toward one another. A biasing element, such as a spring, can optionally be provided to bias the positioning element to an extended position, in which the expandable elements 310, 312 are spaced apart from one another.

In use, the positioning element 314 can be configured to axially contract and radially expand relative to the longitudinal axis 334 upon application of electrical energy to the positioning element 314. Such contraction is effective to decrease a length I of the positioning element 314, thereby sliding the moveable portion 317 proximally relative to the fixed portion 316, and decreasing the distance between the second expandable element 312 on the fixed portion 316 and the first expandable element 314 on the moveable portion 317. As a result, tissue engaged by the first expandable element 310 will be moved toward tissue positioned adjacent to or engage by the second expandable element 312.

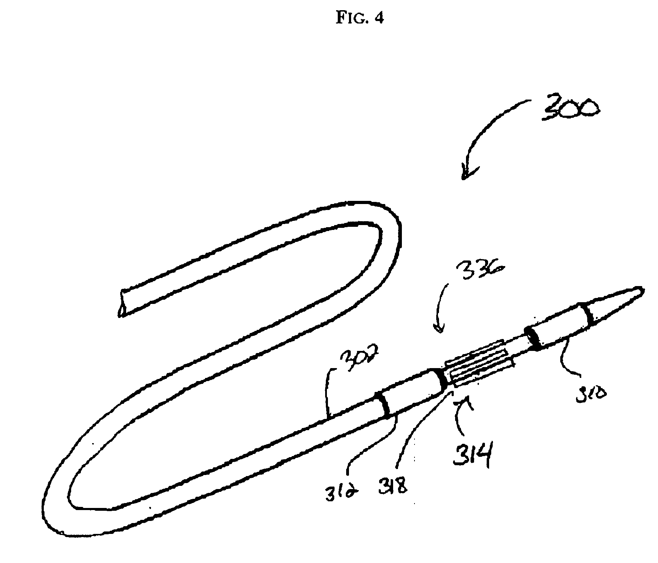

As indicated above, the positioning element 314 can be formed using either the laminate or fiber bundle type EAP. FIG. 4 illustrates another embodiment of a positioning element 314 that is formed using the laminate or composite type EAP which is used to forms EAP bands. While the bands 318 can be disposed within the lumen 305 of the elongate shaft 302 or they can be integrally formed with the elongate shaft 302, in the illustrated embodiment multiple EAP bands 318 are axially disposed along an external surface of the elongate shaft 302. The bands 318 are operable to axially contract upon application of electrical energy thereto. Accordingly, a portion of the elongate shaft 302 extending between the first and second positioning elements 310, 312 can be formed from a flexible material, or can otherwise have a configuration that allows the bands 318 to axially contract the elongate shaft 302. The arrangement of the bands 318 relative to the elongate shaft 302 can vary, but in one embodiment the bands 318 can be spaced substantially equidistant from one another around a circumference of the flexible material 336 of the elongate member 302 to uniformly contract the flexible portion of the elongate shaft 302. In particular, equidistant spacing of the bands 318 will minimize bending or kinking of the flexible material 336 when the bands 318 are electrically activated. The bands 318 can alternatively be configured to bend and/or flex the elongate shaft 302 in a desired direction.

As indicated above, the tissue apposition device 300 can be used to position tissues in apposition to each other, for example to position a stomach pouch in proximity to a small intestine to allow surgical coupling of the tissues and to form an end-to-end anastamosis during a gastric bypass procedure. FIGS. 5A-5F illustrate one exemplary method for positioning and securing a stomach pouch and a small intestine during such a procedure. A person skilled in the art will appreciate that the device 300 can be used in a variety of other medical procedures.

To connect the small stomach pouch with the patient's small intestine, an opening 350 can be formed in a wall 352 of the small stomach pouch and an opening 354 can be formed of a wall 356 of the patient's small intestine, such as by using a tissue cutting device. The openings 350, 354 allow insertion of the tissue apposition device 300 within the stomach pouch and small intestine for apposition of the organs. As shown in FIG. 5A, the first and second expandable elements 310, 312 are in a radially contracted (e.g., non-electrically activated) state, and the tapered tip 320 of the tissue apposition device 300 is used to guide the elongate member 302, in a direction indicated by arrow 358, into and through the openings 350, 354 of the tissue walls 352, 356 to position the first expandable element 310 on an opposed side of the opening 354 in the small intestine wall 356.

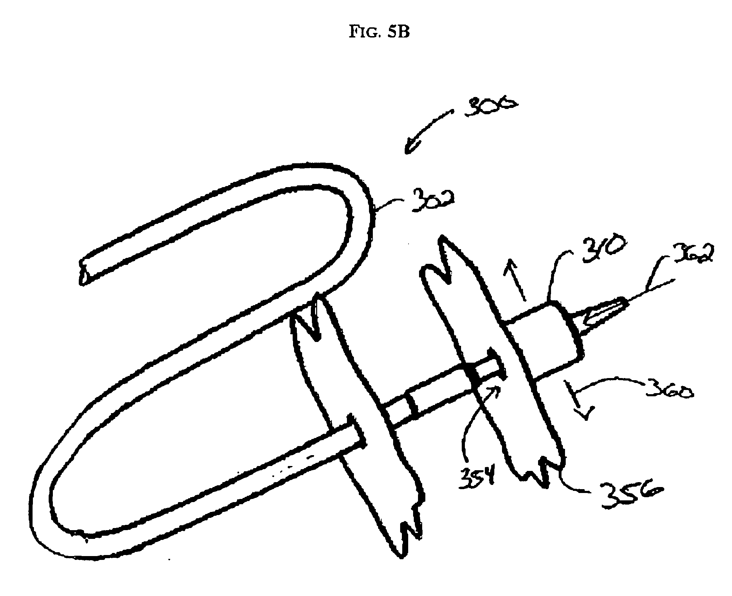

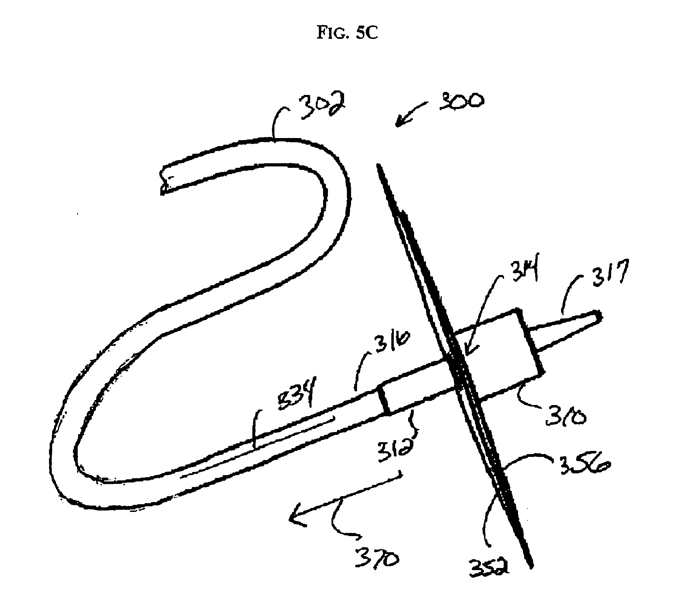

As shown in FIG. 5B. once the first expandable element 310 is inserted into the small intestine, electrical energy can be delivered, e.g., using a button, knob, or dial formed on the handle 304, to the first expandable element 310 to cause a change in the geometry of the first expandable element 310, and more preferably to radially expand the first expandable element 310 to a size that is sufficient to engage the tissue 356, i.e., to prevent passage of the first expandable element 310 through the opening 354 in the small intestine wall 356. The amount of energy delivered can be controlled to expand the first expandable element 310 to a desired size. Once the small intestine wall 356 is engaged, electrical energy can be delivered to the positioning element 314 to change the geometry of the positioning element 314, and in particular to axially contract the positioning element 314 along the longitudinal axis 334 of the elongate member 308, thereby sliding the moveable portion 317 proximally within the fixed portion 316 of the elongate member 302 and moving the first expandable element 310 toward the second expandable element 312, as shown in FIG. 5C. As a result, the small intestine wall 356 is moved into proximity to the stomach pouch wall 352.

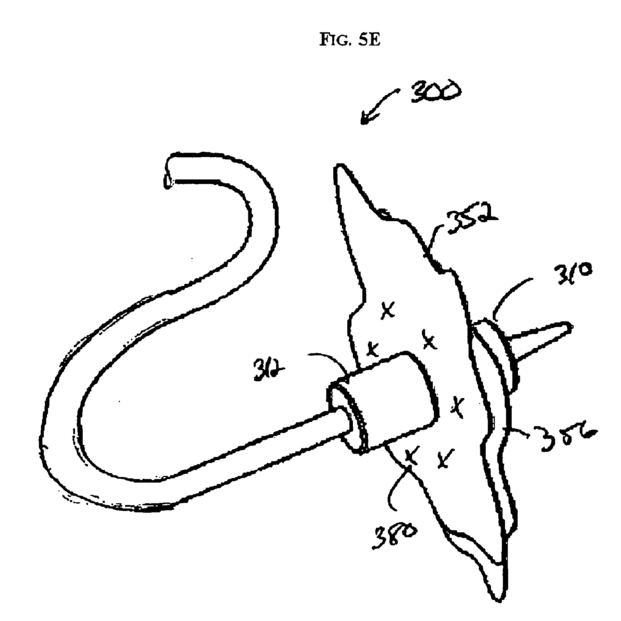

With the small intestine wall 356 positioned in proximity to the stomach pouch wall 352, electrically energy can be delivered to the second expandable element 312 to cause a change in the geometry of the second expandable element 312, and in particular to cause the second expandable element 312 to radially expand and engage the stomach pouch wall 352, thereby securing the stomach pouch wall 352 in proximity to or against the small intestine wall 356. Electrical energy delivery to the expandable elements 310, 312 and the positioning element 314 is preferably maintained to maintain these elements in the electrically actuated state. With both of the expandable elements 310, 312 in a radially expanded state and the positioning element 314 in a contracted state, as shown in FIG. 5E, fasteners 380 such as staples or sutures, can be applied to the small intestine wall 356 and the stomach pouch wall 352 to secure the small intestine and stomach pouch to each other to form an end-to-end anastomosis. For example, staples can be applied to the stomach pouch wall 352 and the stomach pouch wall 352, about the circumference of the second expandable element 312 via a surgical stapler.

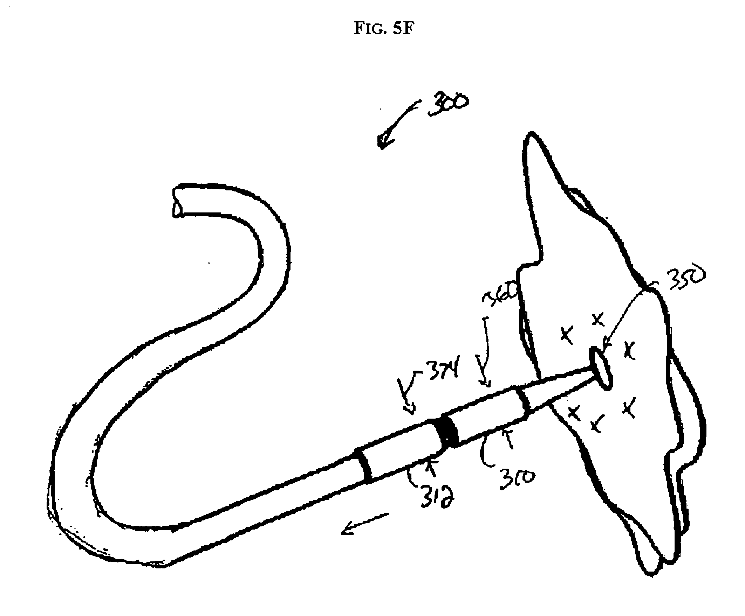

Once the stomach pouch and the small intestine have been secured to each other, the tissue apposition device 300 can be withdrawn from the openings 350, 354 in the tissues. As shown in FIG. 5F, to allow withdrawal of the device 300 from the openings 350, 354, electrical energy delivery to at least the first and second expandable elements 310, 312 can be terminated to cause the first and second expandable elements 310 to radially contract, thereby allowing the the tissue apposition device 300 to be withdrawn from the small intestine wall 356 and the stomach pouch wall 352.

One skilled in the art will appreciate further features and advantages of the invention based on the above-described embodiments. Accordingly, the invention is not to be limited by what has been particularly shown and described, except as indicated by the appended claims.

## Claims

1. A tissue apposition device (300), comprising:
an elongate member (302) having at least two expandable elements (310, 312) coupled thereto; and
a positioning element (314) coupled to the elongate member and adapted to axially move the at least two expandable elements relative to one another;
**characterised in that** the positioning element and the at least two expandable elements are formed from electrically actuatable material and adapted to change dimensionally upon delivery of electrical energy thereto.

2. The tissue apposition device of claim 1, wherein the positioning element is adapted to contract axially when electrical energy is delivered thereto.

3. The tissue apposition device of claim 1, wherein at least one of the expandable elements is adapted to expand radially when electrical energy is delivered thereto.

4. The tissue apposition device of claim 1, wherein the elongate member includes a fixed portion (316) and a moveable portion (317) moveably coupled to the fixed portion, and the at least two expandable elements are coupled respectively to the moveable portion, and the fixed portion.

5. The tissue apposition device of claim 4, wherein the moveable portion is slidably disposed within an inner lumen (305) formed in at least a portion of the fixed portion.

6. The tissue apposition device of claim 4, wherein the positioning element is coupled to and extends between the fixed portion and the moveable portion of the elongate member.

7. The tissue apposition device of claim 4, wherein the positioning element comprises an electroactive polymer cord.

8. The tissue apposition device of claim 7, wherein the electroactive polymer cord comprises a flexible conductive outer shell having an electroactive polymer and an ionic fluid disposed therein.

9. The tissue apposition device of claim 7, wherein the electroactive polymer cord comprises an electroactive polymer composite having at least one flexible conductive layer, an electroactive polymer layer, and an ionic gel layer.

10. The tissue apposition device of claim 4, wherein the moveable portion and the fixed portion are moveably coupled to one another by a flexible portion of the elongate member.

## Patentansprüche

1. Gewebeappositionsvorrichtung (300), die aufweist:
ein längliches Bauteil (302), das wenigstens zwei aufweitbare Elemente (310, 312) aufweist, die an dieses gekoppelt sind; und
ein Positionierelement (314), das an das längliche Bauteil gekoppelt ist und dazu ausgelegt ist, die wenigstens zwei aufweitbaren Elemente relativ zueinander axial zu bewegen;
**dadurch gekennzeichnet, dass** das Positionierelement und die wenigstens zwei aufweitbaren Elemente aus elektrisch betätigbarem Material gebildet sind und so ausgelegt sind, dass sie sich bei Zufuhr elektrischer Energie dimensionsmäßig ändern.

2. Gewebeappositionsvorrichtung nach Anspruch 1, bei der das Positionierelement so ausgelegt ist, dass es sich axial zusammenzieht, wenn elektrische Energie zugeführt wird.

3. Gewebeappositionsvorrichtung nach Anspruch 1, bei dem wenigstens eines der aufweitbaren Elemente so ausgelegt ist, dass es sich radial aufweitet, wenn elektrische Energie zugeführt wird.

4. Gewebeappositionsvorrichtung nach Anspruch 1, bei der das längliche Bauteil einen festen Teilbereich (316) und einen bewegbaren Teilbereich (317), der an den festen Teilbereich beweglich gekoppelt ist, umfasst, und dass die wenigstens zwei aufweitbaren Elemente jeweils an den bewegbaren Teilbereich und an den festen Teilbereich gekoppelt sind.

5. Gewebeappositionsvorrichtung nach Anspruch 4, bei der der bewegbare Teilbereich verschieblich innerhalb eines inneren Lumens (305) angeordnet ist, das in wenigstens einem Teil des festen Teilbereiches gebildet ist.

6. Gewebeappositionsvorrichtung nach Anspruch 4, bei der das Positionierelement an das längliche Bauteil gekoppelt ist und sich zwischen dem festen Teilbereich und dem bewegbaren Teilbereich erstreckt.

7. Gewebeappositionsvorrichtung nach Anspruch 4, bei dem das Positionierelement ein Kabel aus einem elektroaktiven Polymer aufweist.

8. Gewebeappositionsvorrichtung nach Anspruch 7, bei der das Kabel aus elektroaktivem Polymer eine flexible leitende äußere Hülle mit einem elektroaktiven Polymer und ein darin angeordnetes ionisches Fluid aufweist.

9. Gewebeappositionsvorrichtung nach Anspruch 7, bei der das Kabel aus elektroaktivem Polymer einen Verbund mit elektroaktivem Polymer aufweist, der wenigstens eine flexible leitende Schicht, eine Schicht aus elektroaktivem Polymer und eine Schicht aus ionischem Gel umfasst.

10. Gewebeappositionsvorrichtung nach Anspruch 4, bei der der bewegbare Teilbereich und der feste Teilbereich durch einen flexiblen Teil des länglichen Bauteils beweglich aneinander gekoppelt sind.

## Revendications

1. Dispositif pour apposition de tissu (300), comprenant :
■ un élément allongé (302) auquel au moins deux éléments expansibles (310,312) sont couplés ; et
■ un élément de positionnement (314) couplé à l'élément allongé et adapté pour déplacer axialement lesdits au moins deux éléments expansibles l'un par rapport à l'autre ;
**caractérisé en ce que** l'élément de positionnement et lesdits au moins deux éléments expansibles sont formés à partir d'un matériau pouvant être commandé électriquement et sont adaptés pour pouvoir subir une variation dimensionnelle quand une énergie électrique leur est délivrée.

2. Dispositif d'apposition de tissu selon la revendication 1, dans lequel l'élément de positionnement est adapté pour se contracter axialement quand une énergie électrique lui est délivrée.

3. Dispositif d'apposition de tissu selon la revendication 1, dans lequel au moins un desdits éléments expansibles est adapté pour s'expanser radialement quand une énergie électrique lui est délivrée.

4. Dispositif d'apposition de tissu selon la revendication 1, dans lequel l'élément allongé comprend une partie fixe (316) et une partie mobile (317) couplée en relation mobile à la partie fixe et lesdits au moins deux éléments expansibles sont couplés respectivement à la partie mobile et à la partie fixe.

5. Dispositif d'apposition de tissu selon la revendication 4, dans lequel la partie mobile est disposée en relation coulissante à l'intérieur d'une lumière interne (305) formée dans au moins une partie de la partie fixe.

6. Dispositif d'apposition de tissu selon la revendication 4, dans lequel l'élément de positionnement est couplé à, et s'étend entre la partie fixe et la partie mobile de l'élément allongé.

7. Dispositif d'apposition de tissu selon la revendication 4, dans lequel l'élément de positionnement comprend un cordon polymère électroactif.

8. Dispositif d'apposition de tissu selon la revendication 7, dans lequel le cordon polymère électroactif comprend une gaine extérieure conductrice souple dans laquelle sont disposés un polymère électroactif et un fluide ionique.

9. Dispositif d'apposition de tissu selon la revendication 7, dans lequel le cordon polymère électroactif comprend un composite polymère électroactif comprenant au moins une couche conductrice souple, une couche de polymère électroactif, et une couche de gel ionique.

10. Dispositif d'apposition de tissu selon la revendication 4, dans lequel la partie mobile et la partie fixe sont couplées en relation mobile l'une par rapport à l'autre par une partie souple de l'élément allongé.
